# EUROPEAN PATENT APPLICATION

(11) **EP 1 967 512 A2**
(43) Date of publication of application: **10.09.2008**
(21) Application number: 07113313.6
(22) Date of filing: 27.07.2007
(51) Int. Cl.: C07C 303/32, C07C 309/31, C11D 1/22

(54) **Solid detergent composition and process to prepare the same**

(30) Priority: 08.03.2007 IN MU04452007
(71) Applicant: Unilever PLC, London Greater London EC4P 4BQ (GB); Unilever N.V., 3013 AL Rotterdam (NL)
(72) Inventor: The designation of the inventor has not yet been filed
(74) Representative: Bristow, Stephen Robert

(57) **Abstract**

A process to prepare solid form of magnesium salt of linear alkyl benzene sulphonic acid which comprises the step of neutralization of linear alkyl benzene sulphonic acids with a magnesium-based alkali in the presence of 3 to 28 % water by weight of the reaction mixture in a high shear mixer.
A solid detergent composition comprising (i) from 5 to 90 % magnesium salt of linear alkyl benzene sulphonic acid and (ii) from 5 to 70 % builder.

## Description

### Technical field

The invention relates to a novel and selective process for preparing solid form of magnesium salt of linear alkyl benzene sulphonic acid at high concentration levels and to detergent composition having very high levels of this active surfactant. The invention more particularly relates to a non-tower process for preparing solid detergent composition which on the one hand gives all the processing convenience and economic advantages of high-active powder processes and on the other hand gives all the advantageous product attributes presently possible with only the tower drying process e.g high active surfactant levels and concomitant formulation flexibility and relatively low bulk density. The invention also relates to a solid detergent composition containing a novel selective combination of surfactants and builders. The compositions and the process to prepare them are suited to the cleaning of fabric or hard surfaces. It is also possible to use the process and product of the invention in personal washing applications like skin and hair cleansing.

### Background and Prior Art

Cleaning compositions either for fabric washing applications or for cleaning hard surfaces generally have soaps or synthetic surfactants as the active ingredients. While soaps have traditionally been used for such purposes, over the last several decades the trend has generally been to use synthetic surfactant based detergent formulations for fabric washing and hard surface cleaning. Synthetic surfactants are made from neutralization of long chain acids available from petroleum processing streams. Advantages of synthetic surfactants are their availability at competitive price and cleaning performance in a wide range of hard water for fabric washing application. One such widely used class of synthetic surfactant is the linear alkyl benzene sulphonates (LAS) especially the sodium salt. Sodium LAS based formulations have been traditionally prepared by neutralization of the linear alkyl benzene sulphonic acid with sodium carbonate solution/caustic or any other basic alkali. After neutralization with soda/caustic other formulation ingredients are added to prepare a uniform/homogeneous slurry. This slurry is then spray dried to give the detergent powder and this process is known as the Tower route. An advantage of the tower route is that sodium LAS can be prepared in relatively high levels of active surfactant levels of up to 50%. The sodium LAS formulation produced by the tower process also tends to have a low bulk density generally in the range of 0.5 to 0.7 g/cm³ and a high rate of dissolution in water, both of which are highly preferred by the consumer. The disadvantage with the tower route is that spray drying is a relatively expensive process.

There have been many attempts in the past to develop non tower processes to prepare synthetic detergent compositions and several of them have been commercialized. GB1369269 (Colgate, 1974) describes a process for the production of anionic detergent by vigourously mixing detergent sulphonic acid with powdered sodium carbonate in a mixer with a cutting arrangement, for example a Lodige ploughshare mixer. Using this process, detergent powders or an extrudable mass can be prepared without the need for any spray drying step. An advantage of this so called dry mixing process (or non-tower process) is that it is relatively inexpensive. Disadvantage of this process is that it has not been possible to prepare very high active surfactant powders. This is because a large amount of excess granular neutralizing agent e.g soda ash or water insoluble particulate fillers e.g. talc, calcium carbonate, china clay, silica etc. are needed to be added in order to produce free flowing powders, granules or extrudable masses. Typically the highest surfactant concentration of sodium LAS that can be prepared by this dry mixing route is up to 35% by weight of the detergent composition. Another disadvantage of this process is that the powders/granules so produced tend to have high bulk density and low rate of dissolution which are not preferred by the consumers. Further it has not been easy to incorporate high levels of liquid actives e.g. non-ionic surfactants in the powders/granules produced by the dry-mixing route.

It has heretofore not been possible to produce high concentration LAS actives and compositions based on LAS in the user-friendly and inexpensive dry mixing process route which produces detergent which have the user desired properties generally associated with products produced by the tower route viz. low bulk density, high rate of dissolution and formulation flexibility in terms of having high active levels and incorporation of liquid actives. The present inventors have developed a process for formation of solid forms of magnesium salt of linear alkyl benzene sulphonic acid involving a highly selective process condition in the non-tower process with which it is possible to alleviate most of the disadvantages of prior art processes and produce product which has advantages not achievable in the past.

US5486317 (P&G, 1996) describes a process for making a detergent particle comprising neutralization of the acid form of an anionic surfactant in a high shear mixer by a stoichiometric excess of finely divided particulate neutralizing agent which may be calcium or sodium salt of the carbonate having an average particle size of less than 5 microns. This publication does not teach use of magnesium based alkali for neutralization of LAS acid.

US4146551 (Lion, 1979) describes a process for producing the magnesium salt of sulphonic acids and sulfuric esters comprising the step of neutralizing the sulphonic acids and sulphuric esters with an aqueous dispersion containing (1) at least one neutralizing agent selected from the group consisting of magnesium oxide and magnesium hydroxide and (2) at least one neutralizing accelerator selected from the group consisting of benzoic acid, citric acid, malic acid, phosphoric acid, polyphosphoric acid and water soluble salts thereof under a pH of not more than approximately 6. This publication teaches aqueous phase reaction of sulphonic acids with magnesium neutralizing agent and so powder/granule forms of the products will have to be prepared using spray drying in the tower route which is not required in the present process.

EP0135920 (Dresser, 1985) describes a process for making alkaline earth metal salt of alkyl/aryl sulfonic acids where the alkaline earth metal oxide used for neutralization is calcium oxide. The reaction is not carried out in the dry mixing condition but in a slurry state where the reactants are dispersed in organic solvents. The present invention does not require the use of organic solvents. The above publication also does not specifically teach neutralization with magnesium compounds.

Indian patent application 225/MUM/2000 (Hindustan Lever Ltd, published in 2003) describes a synergistic abrasive cleaning composition containing selective combination of surfactants and a process for producing the same. The process comprises neutralization of at least 40% of the acid precursor of the anionic surfactant using at least one mineral of the dolomites group and mixing abrasives and other conventional ingredients such that the total amount of surfactant is between 0.5 and 35% and the amount of abrasives is 30-95% and processing the mixture in a regular manner. Dolomite is a mineral having the chemical formula CaMg(CO₃)₂^{·} The present inventors have found that using dolomite it is not possible to get higher than 35% active surfactant level which can be processed in the form of powders/granules or extruded into bars while with the process of the present invention it is possible to get surfactant levels as high as 90%. Further this published Indian patent application uses high levels of water outside the selective range of water over which the present invention can be worked for preparation of salt of LAS.

### Objects of the Invention

It is thus an object of the present invention to provide for a process to prepare solid form of magnesium salt of linear alkyl benzene sulphonic acid and high active detergent compositions using a simple and cost effective dry mixing route.

It is another object of the present invention to provide for a process to prepare detergent solids using a simple dry mixing route which does not require use of high levels of water-insoluble inorganic fillers which are traditionally necessary in such processes to get products in the powder/granule form or in a form capable of being extruded into bars.

It is yet another object of the present invention to provide for detergent powders/granules which are prepared by the dry mixing route which have high rate of dissolution and relatively low bulk density.

### Summary of the Invention

According to one aspect of the present invention there is provided a process to prepare solid form of magnesium salt of linear alkyl benzene sulphonic acid which comprises the step of neutralization of linear alkyl benzene sulphonic acids with a magnesium based alkali in the presence of 3 to 28% water by weight of the reaction mixture in a high shear mixer.

According to another aspect of the present invention there is provided a solid detergent composition comprising
(i) from 5 to 90 % magnesium salt of linear alkyl benzene sulphonic acid; and
(ii) from 5 to 70 % builder.

### Detailed description of the Invention

The invention will now be described in greater detail. All percentages herein are by weight, calculated on the total composition unless specifically mentioned otherwise.

The invention is directed to manufacture of solid form of magnesium salt of linear alkyl benzene sulphonic acid. By solid form is meant the powder or granule form or in the form of an extrudable bar.

The invention is highly selective in the neutralizing agent which is used for neutralization of the linear alkyl benzene sulphonic acids in that only magnesium based alkalis have been found to give this unique combination of processing convenience and desired product attributes. Other alkali and alkaline earth metal compounds have been used in the past but each one of them has certain drawbacks which are overcome by using the selective magnesium based alkalis of the present invention. With the use of alkali metal compounds of sodium or potassium the highest active levels that has been possible to make has been in the range of 35 to 40%. The sodium or potassium LAS obtained is at best a pasty mass and high levels of inorganic fillers are needed to get the product in a free flowing powder/granule form or to enable the product to be extruded into bars. The present inventors have also tried other alkaline earth metal compounds like those of calcium like calcium hydroxide, carbonate or oxide. It has been determined that amount of the calcium containing neutralizing agent that is needed is far in excess of stoichiometric, generally in the range of 5 to 10 times of stoichiometric requirement in order to get the desired conversion. The present inventors have also tried other metals compounds like those of zinc and aluminium but these neutralizing agents produce similar or different processing difficulties. Surprisingly, with the use of magnesium based alkalis for neutralization of the LAS acid, excellent conversion could be obtained with as little as 5-100% stoichiometric excess of the magnesium based alkali, more preferably with as little as 5-25% stoichometric excess. Even after arriving at the selection of magnesium based alkali, the inventors had to search assiduously to arrive at a window of operation over which most of the disadvantages of the prior art are overcome. It was found that the product in the desired powder/granule or extrudable bar form could be obtained only when the reaction was carried out in a narrow window of water content. The water content during the reaction should be from 3% to 28% of the weight of the reaction mass.

The process disclosed herein is to prepare a solid form of magnesium salt of linear alkyl benzene sulphonic acid comprising the step of neutralising linear alkyl benzene sulphonic acid with a magnesium based alkali in the presence of 3 to 28% water in a high shear mixer.

The structure of linear alkyl benzene sulphonic acid is given below

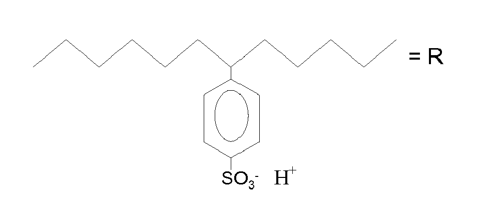

Linear alkylbenzene sulphonic acid is a multi component system of different alkyl chain lengths (R) and position of substitution of the ring in the alkyl chain. The chain length distribution generally ranges from C9 to C13 or C14.

The linear alkyl benzene sulphonic acid is preferably used in the process in amounts in the range of 5 to 90%, more preferably in the range of 10 to 80%, and most preferably 20 to 50% by total weight of reaction mixture.

The magnesium based alkali may be magnesium carbonate, magnesium bicarbonate, magnesium oxide, magnesium hydroxyl carbonate or magnesium hydroxide, more preferably magnesium carbonate, magnesium hydroxyl carbonate or magnesium oxide. The preferred amounts of magnesium based alkali that is added to the reaction mixture is in amounts in the range of 1.5 to 45%, more preferably 2 to 25% by weight of the reaction mixture.

The reaction is carried out essentially in the dry mixing state i.e the amount of water added is very low. The amount of water added is in the range of 3 to 28%, more preferably 4 to 20%, most preferably 4 to 15% by weight of the reaction mixture. A different amount of water needs to be added depending on the amount of LAS acid used and the neutralising agent used. It also depends on the type of product that is desired i.e whether a powder or a granule is desired or whether it has to be extruded into a bar form. In processes of the prior art a large amount of water-insoluble filler materials are added to get the product in the solid form. No water insoluble inorganic filler is required to get either a free flowing powder or granule or an extrudable mass. However up to 3% of water insoluble material may be added. Suitable water insoluble inorganic fillers are talc, calcium carbonate, china clay, silica, sodium aluminosilicate, zeolite, calcium phosphate, calcium silicate, dolomite, magnesium phosphate, magnesium silicate or a clay. However, much higher amounts of water insoluble inorganic fillers may be added at a later stage of detergent composition to get other benefits. The process does not require water outside the range disclosed to enable formation of the solid product form of the detergent active. Additionally the process does not require presence of organic solvents, and ideally no organic solvent is present during the process.

It has been found that the unique nature of magnesium based alkali is that the amount needed for neutralization of LAS acid is minimal as compared to all other neutralising agents tried. The reaction proceeds to completion when as little as 5 to 100 mol% stoichiometric excess of magnesium based alkali is used for reaction with linear alkyl benzene sulphonic acid. The more preferred range is 5 to 25 mole % stoichiometric excess of magnesium based alkali.

The reaction is carried out in a high shear mixer, preferably with a kneading action. The preferred mixers include ploughshare mixer, mixers with kneading members of sigma type, or multi wiping overlap, single curve or double arm. The double arm kneading mixers can be of overlapping or tangential in design. The high shear kneader mixers are provided with cutters to enable reduction of lump formation. Alternatively the process can be carried out in a helical screw agitator vessel.

The process requires minimal energy input. The reactants can be mixed at room temperature i.e anywhere from 10 to 50 °C. Suitable reaction time is from 5 to 60 minutes.

LAS acid is known to be highly corrosive and therefore the neutralization of the LAS acid to the active surfactant is generally carried out in factories located close to the petrochemical producing locations. With the known processes in the prior art, it has not been possible to prepare active surfactant concentrate at higher than 35 to 40% concentration. The general practice has been to form the sodium salt of LAS acid at about 35 to 40% active concentration in factories close to the petrochemical locations. These 35 to 40% surfactant concentrates are then transported to detergent formulation factories which may be geographically distant from the petrochemical factories.

Thus there has been a lot of energy and cost spent in transporting the balance 60 to 65% of the surfactant concentrates which may be water or inorganic fillers which adds to the cost to the consumer. With the process of the present invention, surfactant concentrates at as high as 85% can be prepared in factories located close to the petrochemicals complexes and then shipped or transported to the detergent formulation factories. This will help in reducing the transportation and therefore the overall cost of the product, in addition to all the advantages already discussed earlier.

Once the magnesium salt of LAS has attained the desired solid form as a mixture of magnesium salt of LAS acid along with any residual magnesium based alkali and water, other optional ingredients may be added.

Thus, according to another aspect of the invention there is provided a process for preparation of a solid detergent composition comprising the step of dry mixing a builder with the magnesium salt of linear alkyl benzene sulphonic acid prepared by the first aspect of the invention. The amount of magneisum LAS in this process is preferably in an amount in the range of 5 to 90 wt% by weight of the solid detergent composition. The process for preparation of the solid detergent composition by dry mixing preferably has 5 to 70 % builder by weight of the solid detergent composition.
The builder is preferably chosen from alkali metal carbonates, bicarbonates, phosphates, citrates, silicates or zeolites, especially Zeolite A. The more preferred builders are alkali metal carbonate, bicarbonate or tripolyphosphate. Preferred alkali metal is sodium.

Since the process of the invention is capable of producing solid detergent active in very high levels, detergent compositions in the form of bars, powders and granules having very high levels of detergent active can be prepared without the need of conventional water insoluble structurants. Thereby, the process of the invention gives very high flexibility to the detergent formulator in adding a wide variety of liquid/pasty detergent additives and benefit agents. It is also possible to add high levels of liquid non-ionic surfactants. The solid detergent composition prepared by the process of the invention can be used to make from as low as 5% to as high as 90 % magnesium salt of linear alkyl benzene sulphonate by weight of the detergent composition.

According to another aspect of the present invention there is provided a solid detergent composition comprising (i) 5 to 90 % magnesium salt of linear alkyl benzene sulphonic acid; and (ii) 5 to 70 % builder. A suitable method to prepare the magnesium salt of linear alkyl benzene sulphonic acid in the detergent composition is by using the process described above. The amount of magnesium salt of linear alkyl benzene sulphonic acid can be suitably varied within this range as per the requirement. For low cost powders and bars for fabric washing application, the amount of magnesium LAS may be in the range of 5 to 15%. Premium laundry powders may have Magnesium LAS anywhere from 15 to 30% by weight of the detergent composition. For hard surface cleaning detergent compositions the magnesium LAS content may be anywhere from 5 to 15% by weight of the composition. The solid detergent composition of the present invention comprises builder which is preferably chosen from alkali metal carbonate, bicarbonate, phosphate, citrate, silicate or zeolites. These builders are preferably present from 5 to 60% , most preferably from 10 to 35% by weight of the detergent composition. Highly preferred builders are alkali metal carbonate or phosphate. Most preferred alkali metal is sodium.

The solid detergent composition comprises a minimum of 2% water. When the process is used to prepare the solid detergent composition not more than 28% water is required to get the product in the desired solid shape and form.
However in the detergent composition, it is possible that higher amounts of water can be added at a later stage and water content of the composition can be as high as 30%.

The most suitable product forms of the solid detergent composition are the powder, granule, tablet or bar form, most preferred being the powder or granule form. When the product is in the powder or granule form, the bulk density of the product is generally less than 0.9 g/cm³.

The solid detergent composition has a rate of dissolution that is desired by the consumers. The values are in the range of 0.5 to 0.8 g/litre in 10 minutes as measured using the hyamine titration method. The method of hyamine titration is given below.

An aqueous solution of the sample (detergent powder/bar) is titrated with a standard cationic active solution (Hyamine 1622) in a two-phase water-chloroform system using a mixed indicator (Disulfine Blue-Dimidium Bromide). Cationic dye is dimidium bromide and anionic dye is disulfine blue. The anionic surfactant forms a salt with the cationic dye (dimidium anionic surfactant salt) which dissolves in the chloroform layer to give the organic layer a pink color. The end point is reached when the Hyamine cation displaces the dimidium cation from the chloroform soluble salt and the pink color leaves the chloroform layer as the dye passes to the aqueous phase. At the end point trace of cationic surfactant disulfine blue salt enters in organic layer where a faint blue tint appears in this layer. Hyamine added in excess forms a salt with the anionic dye disulphine blue, which dissolves in the chloroform layer and colors it blue.

The solid detergent composition is substantially free of organic solvents.

It is possible to incorporate high levels of liquid/pasty actives into the solid detergent composition. Liquid/pasty actives include non-ionic surfactants and anionic surfactants like alpha olefin sulphonates (AOS) and primary alkyl sulphates (PAS).

Suitable nonionic detergent surfactant compounds can be broadly described as compounds produced by the condensation of alkylene oxide groups, which are hydrophilic in nature, with an organic hydrophobic compound which may be aliphatic or alkyl aromatic in nature. The length of the hydrophilic or polyoxyalkylene radical which is condensed with any particular hydrophobic group can be readily adjusted to yield a water-soluble compound having the desired degree of balance between hydrophilic and hydrophobic elements.

Particular examples include the condensation product of aliphatic alcohols having from 8 to 22 carbon atoms in either straight or branched chain configuration with ethylene oxide, such as a coconut oil ethylene oxide condensate having from 2 to 15 moles of ethylene oxide per mole of coconut alcohol; condensates of alkylphenols whose alkyl group contains from 6 to 12 carbon atoms with 5 to 25 moles of ethylene oxide per mole of alkylphenol; condensates of the reaction product of ethylenediamine and propylene oxide with ethylene oxide, the condensate containing from 40 to 80% of polyoxyethylene radicals by weight and having a molecular weight of from 5,000 to 11,000; tertiary amine oxides of structure R₃NO, where one group R is an alkyl group of 8 to 18 carbon atoms and the others are each methyl, ethyl or hydroxyethyl groups, for instance dimethyldodecylamine oxide; tertiary phosphine oxides of structure R₃PO, where one group R is an alkyl group of from 10 to 18 carbon atoms, and the others are each alkyl or hydroxyalkyl groups of 1 to 3 carbon atoms, for instance dimethyldodecylphosphine oxide; and dialkyl sulphoxides of structure R₂SO where the group R is an alkyl group of from 10 to 18 carbon atoms and the other is methyl or ethyl, for instance methyltetradecyl sulphoxide; fatty acid alkylolamides; alkylene oxide condensates of fatty acid alkylolamides and alkyl mercaptans. The nonionic can also be selected from a range of alkyl poly glucosides.

It is possible to have higher levels of nonionic surfactant in the detergent composition as compared to sodium LAS based formulations, without compromising on the desirable product properties like free flow in powders.

### Other optional ingredients

The solid detergent composition may comprise any other anionic surfactant which may be alkali or alkaline earth metal, alkanolamine or ammonium salts of precursor acids, or may comprise cationic amphoteric or zwitterionic surfactants to provide specific functionalities. The total amount of such optional synthetic surfactants will generally be up to a maximum of 35% of the composition.

It is also possible optionally to include amphoteric, cationic or zwitterionic detergent surfactants in the compositions.

Further examples of suitable detergent-surfactant compounds are compounds commonly used as surface-active agents given in the well-known textbooks "Surface Active Agents", Volume I by Schwartz and Perry and "Surface Active Agents and Detergents", Volume II by Schwartz, Perry and Berch.

When the detergent composition is used for hard surface cleaning, abrasives are generally added to the detergent composition. Suitable abrasives are selected from, particulate zeolites, calcites, dolomites, feldspar, silicas, silicates, other carbonates, aluminas, bicarbonates, borates, sulphates and polymeric materials such as polyethylene. Preferred abrasives for use in general purpose compositions have a Moh hardness 2-7 although higher hardness abrasives can be employed for specialist applications. Preferred average particle sizes for the abrasive fall in the range 0.5-400 microns preferably 10-200 microns. Preferred levels of abrasive range from 30 to 85wt% of the composition.

The composition can contain other ingredients that aid in their cleaning performance. Composition can also contain, in addition to the ingredients already mentioned, various other optional ingredients such as structurants, colourants, whiteners, optical brighteners, soil suspending agents, detersive enzymes, compatible bleaching agents (particularly hypohalites), and preservatives.

The invention will now be illustrated with respect to the following non-limiting examples.

### Examples:

### Example-1 and Comparative Examples A to C

### Experimental Procedure

A batch of 5 kg of salt of LAS acid was prepared in a Sigma mixer using amount of reactants as shown in Table-1. The process involved taking the indicated amount of metal carbonate in the mixer at about 25°C. After one minute of mixing, LAS acid was added slowly onto the powder with the Sigma mixer blades running to bring it to a homogeneous mixture. After all the LAS acid was added, indicated amount of water was added under mixing to enable neutralization of the LAS acid. The total time for neutralization was about 10 minutes. The temperature of the neutralized mass was in the range of 40 to 65°C. The mixer was run for a further ten minutes to get the final product. No fillers like calcite, talc, clays, silica or china clay which are traditionally used for producing processable products like extrudable mass, powder or granule were used in any of these examples. The experiments were carried out at conditions close to the highest concentrations of detergent actives at which solid products could be prepared. The amount of detergent active produced was measured and the product form noted.

**Table-1**

| Examples | Example -1 | Comparative Example- A | Comparative Example - B | Comparative Example - C |
|---|---|---|---|---|
| LAS Acid (wt%) | 80.5 | 32.2 | 32.7 | 33.1 |
| Metal Carbonate | Magnesium carbonate | Sodium carbonate | Calcium carbonate | Dolomite* |
| Carbonate (wt%) | 15.4 | 69 | 66.6 | 66.3 |
| Stoichiometric amount of carbonate required (Wt%) | 14 | 9 | 6.6 | 6.3 |
| % stoichiometric excess | 10 | 660 | 910 | 870 |
| Water (wt%) | 8.1 | 3 | 4.3 | 4.3 |
| Detergent active (wt%) | 82.2 | 33 | 33 | 33 |
| Product form | Powder | Dough | Dough | Dough |
| Product characteristic | Free flowing and non-hygroscopic | Not extrudable | Not extrudable | Not extrudable |

| | | | | |
|---|---|---|---|---|
| * Dolomite is a natural mined material having the structure CaMg(CO₃)₂. | | | | |

The data in Table-1 for Example - 1 indicates that solid detergent active can be prepared in very high concentrations (>80%) using magnesium based alkali for netralisation of the LAS acid in a high shear mixer. The amount of neutralizing agent (magnesium carbonate) required is minimal (only 10% stoichiometric excess) and a highly user friendly product as free flowing powder is achieved. The data for Comparative examples A, B and C indicates that not more than 35 wt% detergent active in solid form can be prepared using any of the known neutralizing agents (soda, calcite or dolomite). Further the amount of neutralizing agent required is several fold that required by stoichiometry to get a solid product, yet the solid product has poor downstream processing properties.

### Examples - 2 to 7 and Comparative Examples D and E: Effect of amount of water

Various products as shown in Table - 2 were prepared using LAS acid and magnesium carbonate using the process as used for Example -1 but varying the amount of water used. The product characteristic was noted and summarized in Table-2.

**Table-2**

| Examples | Wt% water | Product form | Product Characteristic | Detergent Active (wt%) |
|---|---|---|---|---|
| Comparative Example - D | 2 | Dough | Acidic pH, partially neutralised | 91.9 |
| Example-2 | 4 | Powder | Free flowing, neutral pH | 89.7 |
| Example-3 | 10 | Powder | Free flowing neutral pH | 82.2 |
| Example-4 | 13 | Granules (small) | Free flowing neutral pH | 80.9 |
| Example-5 | 15.6 | Granules (big) | Free flowing neutral pH | 80.5 |
| Example-6 | 20 | Dough (hard) | Free flowing neutral pH | 73.2 |
| Example-7 | 25 | Dough (soft) | Free flowing neutral pH | 68.5 |
| Comparative Example - E | 30 | Dough (very soft) | Not extrudable to bar | 65.8 |
| Comparative Example - F | 36 | Pasty mass | ** | 62.7 |

| | | | | |
|---|---|---|---|---|
| ** Product was not in solid form. The product required large amount of fillers (> 50%) in order to get product which could be extruded. | | | | |

The data in Table - 2 indicates that a selective amount of water in the range of 3 to 28 weight % is necessary in order to prepare magnesium salt of LAS acid through the dry mixing route.

### Comparative Example - G and Examples 8 to 12: Effect of amount of magnesium carbonate used for neutralization

Several experiments were conducted to study the effect of amount of neutralizing agent magnesium carbonate on the product characteristics. The process as described for Example-1 was used for these experiments with the water content added at 8% and the detergent active amounts at 82.2weight %. The results are summarized in Table- 3. Comparative examples A to C are reproduced in this table for comparison with the results obtained as per the examples within the invention (Examples 8 to 12).

**Table-3**

| Examples | Neutralising agent | Mole% neutralizing agent more than stoichiometric | Product form | Product Characteristic |
|---|---|---|---|---|
| Comparative Example - G | Magnesium carbonate | 1 | dough | Acidic pH, partially neutralised |
| Example-8 | Magnesium carbonate | 5 | Powder | Free flowing, |
| Example-9 | Magnesium carbonate | 10 | Powder | Free flowing |
| Example-10 | Magnesium carbonate | 50 | Powder | Free flowing |
| Example-11 | Magnesium carbonate | 100 | Powder | Free flowing |
| Example-12 | Magnesium carbonate | 200 | Powder | Free flowing# |
| Comparative Example - A | Sodium Carbonate | 660 | Dough | Not extrudable |
| Comparative Example - B | Calcium carbonate | 910 | Dough | Not extrudable |
| Comparative Example - C | Dolomite | 870 | Dough | Not extrudable |

| | | | | |
|---|---|---|---|---|
| **#** Good product but such high quantity of MgCO₃ not required. Adds to cost | | | | |

The data in Table-3 indicates that very reasonable amounts of magnesium carbonate (in the range of 5 to 100 mole% over and above that required for stoichiometry in neutralization with LAS acid) are required for preparing magnesium salt of LAS acid in solid form that is easily processible. In comparison other cations as neutralizing agents (Comparative Examples A to C) perform very poorly.

### Comparative Examples H and I and Examples 13 to 17:

Many samples of magnesium salt of LAS acid were prepared at various conditions to study the hygroscopic characteristics of the products produced. The samples were prepared using the process as described for Example-1 and the results are summarized in Table-4. The results are compared to two existing commercial samples viz. Surf Excel powder (Comparative Example H) and Wheel Powder (Comparative Example I).

**Table- 4**

| Examples | Mole% magnesium carbonate above stoichiometric | Product form | Moisture content on manufacture (wt%) | Mositure content on storage at HH condition^{&} |
|---|---|---|---|---|
| Example-13 | 10 | Powder | 9.1 | 8.8 |
| Example-14 | 25 | Powder | 7.5 | 7.5 |
| Example-15 | 50 | Powder | 6.2 | 7.2 |
| Example-16 | 50 | Small granules | 8.5 | 8.0 |
| Example-17 | 50 | Big granules | 8.3 | 7.9 |
| Comparative Example - H | - | Powder | 4.9 | 4.6 |
| Comparative Example - I | - | Powder | 3.0 | 3.0 |

| | | | | |
|---|---|---|---|---|
| ^{&} HH condition: is at 40 degree C and 96% relative humidity | | | | |

The samples of Examples 13 to 17 were free flowing powder/granules after storage at HH condition and comparable to the commercial samples of Surf Excel and Wheel brands. The data in Table-4 indicates that the solid detergent active powder/granule samples prepared as per the invention are not hygroscopic and are acceptable for preparing detergent formulations which can be marketed and get high consumer acceptance.

### Examples 18 to 27 and Comparative Example J and K:

Properties of detergent compositions of the invention as compared to commercial samples

Various magnesium LAS based powder and granule detergent compositions were prepared using the process of Example - 1 and the compositional details are shown in Table-5 (Examples 18 to 27) and properties like the bulk density and solubility were measured. The data is compared against properties of commercially available sodium LAS based formulations Surf Excel (Comparative Example J) and Wheel (Comparative Excel K) .

**Table- 5**

| Example | LAS Active, wt% | Product Form | Water, wt% | Fillers wt% | Builders wt% | Electrolytes wt% | Bulk density |
|---|---|---|---|---|---|---|---|
| Ex - 18 | 82.2 | Powder | 11.1 | 1.4 | 0 | 5.3 | 0.738 |
| Ex - 19 | 81.0 | Granules (1-3 mm) | 12.4 | 1.4 | 0 | 5.3 | 0.503 |
| Ex - 20 | 80.5 | Granules (3 - 5 mm) | 12.9 | 1.4 | 0 | 5.2 | 0.518 |
| Ex - 21 | 22.5 | Powder | 2.5 | 0 | 40 | 35 | 0.875 |
| Ex - 22 | 22.5 | Granules (1-3 mm) | 2.5 | 0 | 40 | 35 | 0.9 |
| Ex - 23 | 22.5 | Granules (3 - 5 mm) | 2.5 | 0 | 40 | 35 | 0.89 |
| Ex - 24 | 27 | Powder | 3 | 0 | 40 | 30 | 0.84 |
| Ex - 25 | 27 | Granules (1-3 mm) | 3 | 0 | 40 | 30 | 0.87 |
| Ex - 26 | 27 | Granules (3 - 5 mm) | 3 | 0 | 40 | 30 | 0.885 |
| Ex - 27 | 65 | Granules (1 -2 mm) | 13.4 | 16 | 0 | 5.6 | 0.64 |
| Comp Ex - J Surf Excel | 24 | - | 3 | 2 | 58.8 | 12.2 | 0.81 |
| Comp Ex - K Wheel | 10 | - | 3 | 16 | 25 | 46 | 1.02 |

For the examples 18 to 27, the filler used was calcite, the builder used was sodium carbonate and the electrolyte was sodium sulphate.

The solubility of the various samples was also measured using the following procedure:
Three grams of the sample was taken and transferred to a 2 litre glass beaker containing 1000 ml water and stirred using an overhead stirrer (with four blades) at 150 rpm and at a temperature of 28°C. The stirring was stopped after 30 minutes and an aliquot of 5 ml was taken immediately and filtered using a 0.45 µm disposable filter. The anionic surfactant content was determined by hyamine titration and solubility was calculated.

The data in Table 5 indicates that various compositions as per the invention (Examples 18 to 27) over a wide range of Mg LAS detergent active concentrations and levels of builders and electrolytes gave product of similar or lower bulk density as compared to Na LAS based commercial samples. Further the solubility of the various samples (examples 18 to 27) was high and comparable to the commercial samples of Comparative Examples J and K having sodium LAS.

### Examples 28 to 41 and Comparative Examples 28A to 41 A

Cleaning performance of various Magnesium LAS based detergent compositions (Examples 28 to 41) were measured using water at various levels of hardness (0 FH, 48 FH and 72 FH) using various test monitors (WFK 10D, WFK 20D, and WFK 30D) which are all test monitors having composite soil on cotton fabric, polycotton and polyester fabric respectively. The cleaning performance of comparative examples (28A to 41A) containing conventional sodium LAS based formulations was also measured. In the experiments with 24 FH and 48 FH, the hardness was from calcium salts while in the case of 72 FH, 48 FH was from Calcium salt and 24 FH from Magnesium salt. The cleaning experiments were as follows:
The detergent composition was dissolved in a tergo-to-meter pot using 90 rpm speed after which the test monitor was soaked for 15 minutes. The test monitor was then washed in the same tergo-to-meter at 90 rpm for 30 minutes. The test monitor was rinsed three times using an L/C ratio (liquor to cloth ratio) of about 25. The test monitor was then dried, after which the reflectance at 460 nm was measured.

The data on cleaning performance in terms of ΔR of the various test monitors of the various examples is summarized in Table - 6. In Table-6, the concentration of the surfactant, the builder (Sodium carbonate) and electrolyte (Sodium sulphate) are given in gpl (grams per litre of the cleaning solution).

**Table- 6**

| **Example** | **FH of Water** | **Test Monitor** | **Surfactant, gpl** | **Builder, gpl** | **Electrolyte, gpl** | **ΔR** |
|---|---|---|---|---|---|---|
| **28** | 0 | 10D | 0.7 | 1.5 | - | **23.1** |
| **28A** | 0 | 10D | 0.7 | 1.5 | - | **24.1** |
| **29** | 0 | 10D | 0.7 | 1.5 | 1.0 | **25.9** |
| **29A** | 0 | 10D | 0.7 | 1.5 | 1.0 | **24.8** |
| **30** | 0 | 20D | 0.7 | 1.5 | - | **25.9** |
| **30A** | 0 | 20D | 0.7 | 1.5 | - | **24.8** |
| **31** | 0 | 20D | 0.7 | 1.5 | 1.0 | **27.4** |
| **31A** | 0 | 20D | 0.7 | 1.5 | 1.0 | **27.4** |
| **32** | **48** | 10D | 0.7 | 1.5 | - | **19.4** |
| **32A** | **48** | 10D | 0.7 | 1.5 | - | **18.6** |
| **33** | **48** | 10D | 0.7 | 1.5 | 1.0 | **19.5** |
| **33A** | **48** | 10D | 0.7 | 1.5 | 1.0 | **19.4** |
| **34** | **48** | 20D | 0.7 | 1.5 | - | **20.0** |
| **34A** | **48** | 20D | 0.7 | 1.5 | - | **18.2** |
| **35** | **48** | 20D | 0.7 | 1.5 | 1.0 | **18.2** |
| **35A** | **48** | 20D | 0.7 | 1.5 | 1.0 | **19.3** |
| **36** | 72 | 10D | 0.7 | 1.5 | - | **15.3** |
| **36A** | 72 | 10D | 0.7 | 1.5 | - | **17.3** |
| **37** | 72 | 10D | 0.7 | 1.5 | 1.0 | **16.8** |
| **37A** | 72 | 10D | 0.7 | 1.5 | 1.0 | **17.9** |
| **38** | 72 | 20D | 0.7 | 1.5 | - | **18.3** |
| **38A** | 72 | 20D | 0.7 | 1.5 | - | **18.6** |
| **39** | 72 | 20D | 0.7 | 1.5 | 1.0 | **19.3** |
| **39A** | 72 | 20D | 0.7 | 1.5 | 1.0 | **18.8** |
| **40** | 72 | 30D | 0.7 | 1.5 | - | **18.6** |
| **40A** | 72 | 30D | 0.7 | 1.5 | - | **16.2** |
| **41** | 72 | 30D | 0.7 | 1.5 | 1.0 | **18.1** |
| **41A** | 72 | 30D | 0.7 | 1.5 | 1.0 | **18.1** |

The data in Table 6 indicates that the cleaning performance of the detergent compositions of the invention (Examples 28 to 41) is comparable to conventional sodium LAS based formulations of the prior art (Comparative Examples 28A to 41A) when used over a very wide range of water hardness on various types of soil fabrics.

The invention thus provides for a process to prepare high active detergent solids using a simple and cost effective dry mixing route. With the use of this novel process it is now possible to prepare detergent solids in the powder, granule or extruded bar forms by the dry mixing route which does not require use of high levels of water-insoluble inorganic fillers.

## Claims

1. A process to prepare solid form of magnesium salt of linear alkyl benzene sulphonic acid which comprises the step of neutralization of linear alkyl benzene sulphonic acids with a magnesium based alkali in the presence of 3 to 28% water by weight of the reaction mixture in a high shear mixer.

2. A process as claimed in claim 1 wherein said solid form is powder, granule or extrudable bar.

3. A process as claimed in claim 1 or claim 2 wherein said acid is neutralized with 5-100% stoichiometric excess, preferably 5 - 25 mol% stoichiometric excess of said magnesium based alkali.

4. A process as claimed in any one of the preceding claims wherein said magnesium based alkali is selected from one or more of magnesium carbonate, magnesium bicarbonate, magnesium oxide, magnesium hydroxycarbonate or magnesium hydroxide.

5. A process as claimed in any one of the preceding claims wherein the process is carried out at a temperature in the range of 10 to 50°C.

6. A process as claimed in any one of the preceding claims wherein the process is carried out for a period of time from 5 to 60 minutes.

7. A process as claimed in any one of the preceding claims wherein less than 3% water insoluble inorganic fillers are added to the reaction mixture to get either a free flowing powder or granule or an extrudable mass.

8. A process as claimed in claim 7 wherein said water insoluble inorganic fillers are selected from one or more of talc, calcium carbonate, china clay, silica, sodium aluminosilicate, zeolite, calcium phosphate, calcium silicate, dolomite, magnesium phosphate, magnesium silicate or a clay.

9. A process for preparation of a solid detergent composition comprising the step of dry mixing a builder with said solid form of magnesium salt of linear alkyl benzene sulphonic acid prepared by a process as claimed in any one of the preceding claims.

10. A process as claimed in claim 9 comprising 5 to 90% magnesium salt of linear alkyl benzene sulphonic acid by weight of the solid detergent composition.

11. A process as claimed in claim 9 or 10 comprising 5 to 70% builder by weight of the solid detergent composition.

12. A process to prepare a solid detergent composition as claimed in any one of the preceding claims 10 to 12 wherein said builder is chosen from alkali metal carbonates, bicarbonates, phosphates, citrates, silicates or zeolites, preferably the builder is an alkali metal carbonate, bicarbonate or tripolyphosphate.

13. A process to prepare a solid detergent composition as claimed in claim 12 wherein said alkali metal is sodium.

14. A solid detergent composition comprising
(i) from 5 to 90 % magnesium salt of linear alkyl benzene sulphonic acid; and
(ii) from 5 to 70 % builder.

15. A solid detergent composition as claimed in claim 16 wherein said builder is an alkali metal carbonate, bicarbonate, phosphate, citrate, silicate or a zeolite, preferably said builder is alkali metal carbonate or phosphate and most preferably said alkali metal is sodium.

16. A solid detergent composition as claimed in any one of the preceding claims 16 to 19 comprising 2 to 30 % water.

17. A solid detergent composition as claimed in any one of preceding claims 16 to 20 in the powder, granule, tablet or bar form, preferably in powder or granule form.

18. A solid detergent composition as claimed in claim 17 which is a powder or granule having bulk density less than 0.9 g/cm³.

19. A solid detergent composition as claimed in any one of claims 14 to 18 wherein the rate of dissolution as determined by the hyamine titration method is in the range of 0.5 to 0.8 g/litre in 10 minutes.

20. A solid detergent composition as claimed in any one of claims 14 to 19, substantially free of organic solvents.
